(19) Europäisches Patentamt

European Patent Office

Office européen des brevets

(11) Veröffentlichungsnummer: **0 604 806 A2**

# (12) EUROPÄISCHE PATENTANMELDUNG

(21) Anmeldenummer: 93119922.8

(22) Anmeldetag: **10.12.93**

(51) Int. Cl.⁵: **A61K 7/48**, A23J 7/00,
A61K 31/66, A61K 31/685

(30) Priorität: **18.12.92 DE 4242959**
**18.12.92 DE 4242960**
**26.01.93 DE 4302074**
**24.02.93 DE 4305554**

(43) Veröffentlichungstag der Anmeldung:
**06.07.94 Patentblatt 94/27**

(84) Benannte Vertragsstaaten:
**AT BE CH DE DK ES FR GB GR IE IT LI LU MC NL PT SE**

(71) Anmelder: **RHONE-POULENC RORER GMBH**
**Postfach 35 01 20,**
**Nattermannallee 1**
**D-50792 Köln(DE)**

(72) Erfinder: **Fussbroich, Peter**
**Suitbertstrasse 71**
**D-51067 Köln(DE)**
Erfinder: **Gareiss, Johannes**
**Herweghstrasse 5**
**D-50829 Köln(DE)**
Erfinder: **Ghyczy, Miklos**
**Im Rapsfeld 23**
**D-50933 Köln(DE)**
Erfinder: **Schwentke, Horst**
**Neusser Landstrasse 110**
**D-50769 Köln(DE)**
Erfinder: **Wendel, Armin**
**Max-Ernst-Strasse 29**
**D-50859 Köln(DE)**
Erfinder: **Wiedemann, Maria**
**Usedomstrasse 52**
**D-50765 Köln(DE)**

(74) Vertreter: **Döring, Wolfgang, Dr. Ing.**
**Mörikestrasse 18**
**D-40474 Düsseldorf (DE)**

(54) **Phospholipidische Zusammensetzung.**

(57) Es wird eine phospholipidische Zusammensetzung beschrieben, die neben üblichen Inhaltsstoffen mindestens 20 Gew.% Phosphatidylcholin, mindestens 20 Gew.% N-Acyl-Phosphatidylethanolamin sowie weniger als 3 Gew.% Phosphatidylethanolamin enthält.

Ein Verfahren zur Herstellung der phospholipidischen Zusammensetzung setzt acyliertes Rohlecithin als Ausgangsmaterial ein und behandelt dieses acylierte Rohlecithin mit Methanol. Nach Abtrennen eines dabei anfallenden Rückstandes wird die phospholipidische Zusammensetzung aus dem Methanol-Extrakt isoliert.

EP 0 604 806 A2

Die vorliegende Erfindung betrifft eine phospholipidische Zusammensetzung mit den Merkmalen des Oberbegriffs des Patentanspruchs 1 sowie ein Verfahren zur Herstellung einer derartigen phospholipidischen Zusammensetzung mit den Merkmalen des Oberbegriffs des Patentanspruchs 11.

Phospholipidische Zusammensetzungen kommen in der Natur mit unterschiedlichem chemischem Aufbau vor. So können beispielsweise phospholipidische Zusammensetzungen aus tierischen Ausgangsprodukten, wie beispielsweise aus Eiern, isoliert werden. Darüber hinaus können pflanzliche Ausgangsmaterialien, wie z.B. Kokosnuß-Kopra, Palmkerne, Erdnüsse, Raps, Sonnenblumenkerne, Ölpalmen, Oliven oder insbesondere Sojabohnen, für die Gewinnung von phospholipidischen Zusammensetzungen eingesetzt werden.

Zur Isolierung der phospholipidischen Zusammensetzung aus den zuvor genannten pflanzlichen Ausgangsmaterialien wird das entsprechende Öl, beispielsweise durch Einleiten von geringen Mengen Wasserdampf oder Wasser, entschleimt. Die dabei entstehende phospholipidische Zusammensetzung, die auch Lecithinschlamm genannt wird, weist in der Regel zwischen etwa 8 Gew.% und 59 Gew.% Phospholipide auf, wobei der Lecithinschlamm anschließend getrocknet wird, so daß nach der Trocknung ein so genanntes Rohlecithin anfällt.

Abhängig von dem jeweils eingesetzten Ausgangsmaterial, aus dem der Lecithinschlamm isoliert wurde, variiert die chemische Zusammensetzung eines derartigen Lecithinschlamms. So besteht das wohl wichtigste Rohlecithin, das aus Sojabohnen isoliert und dementsprechend auch als Sojalecithin benannt wird, gemäß PARDUN (Pardun, H., Die Pflanzenlecithine, Verlag für Chem. Industrie H. Ziolkowsky, 1988) aus etwa

30 Gew.% - 35 Gew.% Triglyceride;
2 Gew.% - 5 Gew.% freie Fettsäuren, Sterine, Tocopherole;
11 Gew.% - 17 Gew.% Glykolipide, Kohlehydrate;
48 Gew.% - 55 Gew.% Phospholipide; und
0,2 Gew.% - 0,7 Gew.% Wasser.

Wie dem zuvor wiedergegebenen chemischen Aufbau zu entnehmen ist, stellen die etwa 48 Gew.% - etwa 55 Gew.% Phospholipide den Hauptbestandteil von phospholipidischen Zusammensetzungen dar. Diese Phospholipide im Rohlecithin bestehen im wesentlichen aus Phosphatidylcholin, Phosphatidylethanolamin und Phosphatidylinosit, wobei die Massen dieser Hauptbestandteile, bezogen auf die Masse des Rohlecithins, in etwa wie folgt variieren:

12 Gew.% - 18 Gew.% Phosphatidylcholin;
9 Gew.% - 15 Gew.% Phosphatidylethanolamin; und
8 Gew.% - 12 Gew.% Phosphatidylinosit.

Neben den zuvor genannten Hauptbestandteilen weist das rohe Sojalecithin Lysophosphatidylcholin, Lysophosphatidylethanolamin, Phosphatidsäure sowie N-Acyl-Phosphatidylethanolamin als weitere phospholipidische Inhaltsstoffe auf, wobei die Konzentration an N-Acyl-Phosphatidylethanolamin zwischen 1 Gew.% und 3 Gew.%, bezogen auf die Masse des rohen Sojalecithins, variiert.

Das zuvor genannte Rohlecithin kann, abhängig von dem jeweiligen Anwendungszweck, weiterverarbeitet werden. So ist es beispielsweise möglich, aus Rohlecithin durch acetonische Entölung ein Reinlecithin herzustellen, wobei bei der acetonischen Entölung die eingangs genannten Neutrallipide aus den Rohlecithinen weitestgehend entfernt werden, so daß der Phosphatidylcholingehalt eines derartigen Reinlecithins auf einen Wert zwischen 25 Gew.% und 30 Gew.%, bezogen auf die Masse des Reinlecithins, ansteigt.

Desweiteren ist es möglich, den Lecithinschlamm, der, wie bereits vorstehend ausgeführt ist, durch Einleiten von geringen Mengen Wasserdampf oder Wasser aus dem Pflanzenöl entsteht, durch Umsetzung mit Säureanhydriden zu acylieren, wobei nach Trocknung acylierte Lecithine entstehen, die im wesentlichen

12 Gew.% - 18 Gew.% Phosphatidylcholin;
3 Gew.% - 5 Gew.% Phosphatidylethanolamin;
6 Gew.% - 10 Gew.% N-Acyl-Phosphatidylethanolamin; und
8 Gew.% - 12 Gew.% Phosphatidylinosit

als phospholipidische Inhaltsstoffe enthalten. Weiterhin weist das acylierte Lecithin noch geringe Konzentrationen der zuvor beim Rohlecithin genannten weiteren Phospholipide auf.

Die zuvor genannten acylierten Lecithine können nur bedingt im pharmazeutischen und kosmetischen Bereich eingesetzt werden. Dies hängt damit zusammen, daß bei Verwendung von acylierten Lecithinen die Gefahr besteht, daß entsprechend hiermit hergestellte kosmetische oder pharmazeutische Zubereitungen relativ schnell altern, was sich beispielsweise in unerwünschten Geschmacks-, Farb- und/oder Geruchsveränderungen der entsprechenden Zubereitungen ausdrücken kann.

Der vorliegenden Erfindung liegt die Aufgabe zugrunde, eine phospholipidische Zusammensetzung zur Verfügung zu stellen, die für die Herstellung von lagerstabilen Lebensmitteln, Genußmitteln, Kosmetika und/oder pharmazeutischen Zubereitungen besonders geeignet ist.

Diese Aufgabe wird erfindungsgemäß durch eine phospholipidische Zusammensetzung mit den kennzeichnenden Merkmalen des Patentanspruchs 1 gelöst.

Die erfindungsgemäße phospholipidische Zusammensetzung mit einem Gehalt von mindestens 20 Gew.% Phosphatidylcholin weist neben den üblichen Inhaltsstoffen desweiteren mindestens 20 Gew.% N-Acyl-Phosphatidylethanolamin sowie weniger als 3 Gew.% Phosphatidylethanolamin auf.

Überraschend konnte festgestellt werden, daß die zuvor genannte erfindungsgemäße phospholipidische Zusammensetzung hervorragend für die Herstellung von Lebensmitteln, Genußmitteln, Kosmetika und/oder pharmazeutischen Produkten eingesetzt werden kann, da die erstellten Lebensmittel, Genußmittel, Kosmetika und/oder pharmazeutischen Produkte eine hohe Lagerstabilität aufweisen, was bei Verwendung der bekannten und eingangs beschriebenen acylierten Phospholipiden nur bedingt möglich ist. Auch treten bei der Verwendung der erfindungsgemäßen phospholipidischen Zusammensetzung keine unerwünschten Geruchs- und/oder Geschmacksveränderungen der hiermit hergestellten Lebensmittel, Genußmittel, Kosmetika und/oder Pharmazeutika auf, was darauf zurückgeführt wird, daß in der erfindungsgemäßen Zusammensetzung bewußt die Konzentration an Phosphatidylethanolamin sehr gering gehalten wird, d.h. sie beträgt weniger als 3 Gew.%. Durch einen derartig geringen Gehalt an Phosphatidylethanolaminen werden unerwünschte Nebenreaktionen zwischen dem Phosphatidylethanolamin und den in den Lebensmitteln, Genußmitteln, Kosmetika und/oder Pharmazeutika enthaltenen Aroma- und/oder Wirkstoffen verhindert, so daß bei Verwendung der erfindungsgemäßen phospholipidischen Zusammensetzung demnach auch keine oder nur geringe unerwünschte und den Geruch und/oder den Geschmack verändernde Reaktionsprodukte von Phosphatidylethanolamin mit Wirk- und/oder Aromastoffen entstehen können. Wegen der nicht oder nur im untergeordneten Maß stattfindenden Reaktionen zwischen dem in der erfindungsgemäßen phospholipidischen Zusammensetzung enthaltenen Phosphatidylethanolamin und den Wirkstoffen bzw. Aromastoffen können diese auch nicht bei der Herstellung und/oder Lagerung der Lebensmittel, Genußmittel, Kosmetika und/oder pharmazeutischen Produkte unwirksam gemacht werden, wodurch die höhere Wirksamkeit und die verbesserte Lagerstabilität der unter Verwendung der erfindungsgemäßen phospholipidischen Zusammensetzung hergestellten kosmetischen und pharmazeutischen Zubereitungen sowie der entsprechenden Lebensmittel bzw. Genußmittel erklärlich wird. Auch treten bei Verwendung der erfindungsgemäßen phospholipidischen Zusammensetzung bei der Herstellung der zuvor genannten Produkte keine unerwünschten Farbveränderungen der Fertigprodukte auf, selbst dann nicht, wenn diese Fertigprodukte über einen längeren Zeitraum lagern. Auch diese Farbstabilität der Fertigprodukte wird darauf zurückgeführt, daß es bei der erfindungsgemäßen phospholipidischen Zusammensetzung zu keiner Reaktion zwischen dem Phosphatidylethanolamin und den entsprechenden Inhaltsstoffen der Lebensmittel, Genußmittel, Kosmetika bzw. pharmazeutischen Zubereitungen kommt. Weiterhin scheint die erfindungsgemäße phospholipidische Zusammensetzung eine unerwünschte Oxidation von damit hergestellten Lebensmitteln, Genußmitteln, Kosmetika und/oder pharmazeutischen Zubereitungen zu verhindern, wobei diese Schutzwirkung auf die hohe Konzentration von N-Acyl-Phosphatidylethanolaminzurückgeführt wird.

Darüber hinaus weist die erfindungsgemäße phospholipidische Zusammensetzung weitere Vorteile auf. So konnte festgestellt werden, daß sich die erfindungsgemäße phospholipidische Zusammensetzung hervorragend als Dispergiermittel bzw. Emulgiermittel eignet, so daß mit der erfindungsgemäßen phospholipidischen Zusammensetzung eine Vielzahl von Inhaltsstoffen bzw. Wirkstoffen von Lebensmitteln, Genußmitteln, Kosmetika und/oder pharmazeutischen Zubereitungen hervorragend dispergiert bzw. emulgiert werden können. Bei entsprechend hergestellten Dispersionen bzw. Emulsionen, bei denen es sich sowohl um Öl-in-Wasser-Emulsionen als auch um Wasser-in-Öl-Emulsionen handelte, treten auch bei extrem langer Lagerzeit keine unerwünschten Entmischungsphänomene auf, so daß beispielsweise entsprechend hergestellte flüssige oder pastenförmige Lebensmittel, Genußmittel, Kosmetika oder pharmazeutische Zubereitungen eine über einen langen Zeitraum gleichbleibende Konsistenz aufweisen. Ebenso ruft die erfindungsgemäße Zusammensetzung bei einer topischen Anwendung selbst bei sehr empfindlichen Benutzern keine Hautreizungen oder Hautrötungen hervor, so daß sich die erfindungsgemäße Zubereitung hervorragend als Grundstoff für die Herstellung von topisch anzuwendenden Kosmetika bzw. Pharmazeutika eignet, wie z.B. Salben, Cremes, Shampoos o.dgl..

Außer dem zuvor genannten Phosphatidylcholin, dem N-Acylphosphatidylethanolamin sowie dem Phosphatidylethanolamin weist die erfindungsgemäße phospholipidische Zusammensetzung als übliche Inhaltsstoffe solche Inhaltsstoffe auf, wie diese vorstehend unter Angabe von Gewichtsverhältnissen genannt sind, nämlich Triglyceride, freie Fettsäuren, Sterine, Tocopherole, Glykolipide, Kohlehydrate und Wasser. Weiterhin enthält die erfindungsgemäße phospholipidische Zusammensetzung noch andere Phospholipide, wie beispielsweise Lysophosphatidylcholin, Lysophosphatidylethanolamin, Phosphatidsäure und/oder Derivate davon.

Bezüglich der Konzentration an Phosphatidylcholin in der erfindungsgemäßen phospholipidischen Zusammensetzung ist festzuhalten, daß grundsätzlich diese Konzentration an Phosphatidylcholin über 20 Gew.%, bezogen auf die phospholipidische Zusammensetzung, betragen soll. Vorzugsweise weist jedoch die phospholipidische Zusammensetzung eine Konzentration an Phosphatidylcholin zwischen 22 Gew.% und 40 Gew.%, insbesondere zwischen 25 Gew.% und 30 Gew.%, bezogen auf die Masse der phospholipidischen Zusammensetzung, auf.

Die Konzentration des N-Acyl-Phosphatidylethanolamins in der erfindungsgemäßen Zusammensetzung variiert zwischen 21 Gew.% und 40 Gew.%, insbesondere zwischen 24 Gew.% und 28 Gew.%.

Um die eingangs bei der erfindungsgemäßen phospholipidischen Zusammensetzung beschriebenen unerwünschten Reaktionen zwischen dem Phosphatidylethanolamin und den Inhaltsstoffen bzw. Wirkstoffen zu verhindern, sieht eine weitere, besonders geeignete Ausführungsform der erfindungsgemäßen phospholipidischen Zusammensetzung vor, daß hierbei die Zusammensetzung zwischen 0,01 Gew.% und 1,5 Gew.% Phosphatidylethanolamin aufweist. Vorzugsweise dann, wenn diese Konzentration an Phosphatidylethanolamin unter 1 Gew.%, bezogen auf die phospholipidische Zusammensetzung, liegt, sind diese unerwünschten Nebenreaktionen, die zu Geruchs- und/oder Geschmacksveränderungen oder zu unerwünschten Veränderungen der Farbe führen können, ausgeschlossen.

Eine andere Ausführungsform der erfindungsgemäßen Zusammensetzung sieht vor, daß hierbei die erfindungsgemäßen Zusammensetzungen mindestens 30 Gew.% negativ geladene Phospholipide aufweist, so insbesondere N-Acyl-Phosphatidylethanolamine in der zuvor genannten Konzentration, zwischen etwa 1 Gew.% und 3 Gew.% Phosphatidsäure sowie zwischen etwa 2 Gew.% und etwa 6 Gew.% Phosphatidylinositol sowie andere negativ geladene Phospholipide, wie vorzugsweise Salze von Phosphatidylglycerol oder entsprechende Derivate.

Bedingt durch die vorstehend angegebene relativ hohe Konzentration an negativ geladenen Phospholipiden eignet sich eine derartige Ausführungsform der erfindungsgemäßen Zusammensetzung hervorragend zur Stabilisierung von Liposomen, die aus solchen Phospholipidfraktionen hergestellt sind, die eine hohe Konzentration an Phosphatidylcholin aufweisen, so insbesondere eine Konzentration von mindestens 80 Gew.% und vorzugsweise 90 Gew.% Phosphatidylcholin.

Eine besonders geeignete und bevorzugt eingesetzte erfindungsgemäße phospholipidische Zusammensetzung weist

25 Gew.% - 30 Gew.% Phosphatidylcholin,

24 Gew.% - 28 Gew.% N-Acyl-Phosphatidylethanolamin,

0,01 Gew.% - 1,5 Gew.% Phosphatidylethanolamin, und

40,5 Gew.% - 50,99 Gew.% sonstige Inhaltsstoffe

auf. Bei den zuvor genannten sonstigen Inhaltsstoffen handelt es sich um die zuvor genannten Inhaltsstoffe, insbesondere um Triglyceride, freie Fettsäuren, Sterine, Tocopherole, Glykolipide, Kohlehydrate oder sonstige Phospholipide wie beispielsweise Phosphatidsäure, Lysophosphatidylethanolamin, Phosphatdiylglycerol und/oder Derivate davon. Die zuvor beschriebene Ausführungsform der erfindungsgemäßen phospholipidischen Zusammensetzung weist ausgezeichnete Dispergier- und Emulgiereigenschaften für eine Vielzahl von Wirkstoffen oder Inhaltsstoffen auf, so daß diese Ausführungsform besonders geeignet ist, beispielsweise für die Herstellung von Lebensmitteln, Genußmitteln, Kosmetika oder pharmazeutischen Zubereitungen eingesetzt werden kann. Daneben besitzt diese Ausführungsform im verstärkten Maße all die Vorteile, die eingangs bei der erfindungsgemäßen phospholipidischen Zusammensetzung ausführlich beschrieben sind.

Vorstehend ist im Zusammenhang mit der erfindungsgemäßen Zusammensetzung allgemein nur von N-Acyl-Phosphatidylethanolamin gesprochen worden. Vorzugsweise handelt es sich bei diesem N-Acyl-Phosphatidylethanolamin um ein N-Oleyl-Phosphatidylethanolamin, ein N-Palmitoyl-Phosphatidylethanolamin, ein N-Stearyl-Phosphatidylethanolamin, ein N-Myristoyl-Phosphatidylethanolamin und/oder ein N-Acetyl-Phosphatidylethanolamin jeweils allein oder in Mischung, wobei insbesondere bevorzugt eine solche erfindungsgemäße phospholipidische Zusammensetzung eingesetzt wird, bei der das N-Acyl-Phosphatidylethanolamin zumindestens aus 90 Gew.% N-Acetyl-Phosphatidylethanolaminen besteht. Eine derartige Ausführungsvariante der erfindungsgemäßen Zusammensetzung läßt sich insbesondere auch zur Herstellung von solchen pharmazeutischen oder kosmetischen Zubereitungen einsetzen, die topisch angewendet werden, da insbesondere das N-Acetyl-Phosphatidylethanolamin eine hohe Wirksamkeit in bezug auf die Verhinderung von Hautreizungen oder Augenreizungen besitzt und zudem noch in bezug auf Hautentzündungen oder Hautschädigungen therapeutisch wirkt, wie dies in den deutschen Patentanmeldungen P 42 42 959.5 und P 42 42 960.9 ausführlich beschrieben ist.

Zuvor sind im Zusammenhang mit der erfindungsgemäßen phospholipidischen Zusammensetzung Konzentrationen insbesondere für Phosphatidylcholin, N-Acyl-Phosphatidylethanolamin und Phosphatidy-

4

lethanolamin sowie für die üblichen Inhaltsstoffe angegeben, wobei sich diese Konzentrationsangaben auf eine feste, unverdünnte phospholipidische Zusammensetzung beziehen. Dies bedeutet, daß die feste phospholipidische Zusammensetzung als solche verwendet wird. Für spezielle Anwendungsfälle bietet es sich jedoch insbesondere auch unter dem Gesichtspunkt der besseren Handhabung an, hier flüssige Formulierungen der phospholipidischen Zusammensetzung zu verwenden, vorzugsweise Lösungen der erfindungsgemäßen phospholipidischen Zusammensetzung. Als Lösungsmittel für die erfindungsgemäße phospholipidische Zusammensetzung kommen abhängig von dem jeweiligen chemischen Aufbau $C_1$-$C_4$-Alkohole, Mono- und/oder Diglyceride und/oder Polyalkohole in Frage, wobei bevorzugt die phospholipidische Zusammensetzung in Propylenglykol gelöst ist. Um derartige Lösungen der erfindungsgemäßen phospholipidischen Zusammensetzung herzustellen, werden 70 Gew.% bis 90 Gew.% der zuvor beschriebenen phospholipidischen Zusammensetzungen, die mindestens 20 Gew.% Phosphatidylcholin, mindestens 20 Gew.% N-Acyl-Phosphatidylethanolamin und weniger als 3 Gew.% Phosphatidylethanolamin enthalten, in 30 Gew.% bis 10 Gew.% Lösungsmittel, insbesondere Propylenglykol, gelöst.

Die vorliegende Erfindung betrifft desweiteren ein Verfahren zur Herstellung der zuvor beschriebenen phospholipidischen Zusammensetzung.

Bei dem erfindungsgemäßen Verfahren zur Herstellung der zuvor beschriebenen phospholipidischen Zusammensetzung wird als Ausgangssubstanz ein acyliertes Rohphosphatid eingesetzt, wobei das acylierte Rohphosphatid mit Methanol behandelt und der in Methanol lösliche Anteil isoliert wird.

Das erfindungsgemäße Verfahren zeichnet sich durch den besonderen Vorteil aus, daß es einfach und mit einem relativ geringen apparativen Aufwand durchzuführen ist. Dies wiederum führt dazu, daß nach dem erfindungsgemäßen Verfahren besonders schnell und extrem kostengünstig die zuvor beschriebene erfindungsgemäße phospholipidische Zusammensetzung herstellbar ist, wobei diese phospholipidische Zusammensetzung dann mindestens 20 Gew.% Phosphatidylcholin, mindestens 20 Gew.% N-Acyl-Phosphatidylethanolamin und weniger als 3 Gew.% Phosphatidylethanolamin sowie sonstige übliche Inhaltsstoffe, wie beispielsweise freie Fettsäuren, Sterine, Tocopherole, Glykolipide, Kohlehydrate, Wasser, sonstige Phospholipide, wie insbesondere Lysophosphatidylcholin, Lysophosphatidylethanolamin, Phosphatidsäure und/oder Phosphatidylglycerol, enthält.

Vorzugsweise wird bei dem erfindungsgemäßen Verfahren als Ausgangssubstanz ein solches acyliertes Rohphosphatid eingesetzt, das durch Acylierung in der bekannten Weise, beispielsweise nach PARDUN ("Die Pflanzenlecithine", Verlag für chemische Industrie H, Ziolkowsky KG, Augsburg, 1988) aus rohem Sojalecithin, hergestellt ist.

Um eine unerwünschte Veränderung des eingesetzten acylierten Rohphosphatids bei der Behandlung mit dem Methanol zu verhindern und um insbesondere sicherzustellen, daß die aus dem acylierten Rohlecithin mit Methanol zu isolierende Fraktion auch den chemischen Aufbau aufweist, wie dieser vorstehend für die phospholipidische Zusammensetzung beschrieben ist, wird vorzugsweise die Behandlung bei Raumtemperatur, d.h. somit in einem Temperaturbereich zwischen 5 °C und 35 °C, insbesondere zwischen 10 °C und 15 °C, durchgeführt. Selbstverständlich ist es jedoch auch möglich, hier höhere Temperaturen, so zum Beispiel Temperaturen zwischen 35 °C und 60 °C, auszuwählen, wobei jedoch die erhöhte Temperatur dann die Gefahr beinhaltet, daß es hierbei zu unerwünschten Nebenreaktionen des acylierten Ausgangsproduktes kommt.

Wird bei dem erfindungsgemäßen Verfahren die Behandlung mit dem Methanol bei einer Temperatur zwischen 5 °C und 35 °C durchgeführt, so variiert die Behandlungszeit zwischen 30 Minuten und 3 Stunden. Eine Temperaturerhöhung auf etwa 50 °C bewirkt eine Verkürzung der Behandlungszeit auf 25 Minuten bis 1,5 Stunden.

Um Reste des bei der Behandlung eingesetzten Methanols aus dem methanollöslichen Anteil zu entfernen, wird dieser Extrakt bei Temperaturen zwischen 70 °C und 120 °C bei Normaldruck getrocknet.

Um eine Reproduzierbarkeit bezüglich des quantitativen chemischen Aufbaus des bei der Behandlung mit Methanol extrahierten Anteils, der die erfindungsgemäße phospholipidische Zusammensetzung darstellt, sicherzustellen, empfiehlt es sich, bei der Herstellung der erfindungsgemäßen phospholipidischen Zusammensetzung ein Massenverhältnis von acyliertem Rohphosphatid zu Methanol auszuwählen, das zwischen 1:3 bis 1:5 variiert.

Wie bereits vorstehend ausgeführt ist, kann die so gewonnene erfindungsgemäße phospholipidische Zusammensetzung entweder im festen Zustand weiterverarbeitet oder vorzugsweise in einem geeigneten Lösungsmittel, insbesondere Propylenglykol, aufgenommen werden. Hierfür wird ein Massenverhältnis des isolierten, in Methanol löslichen Anteils (methanolfreie, erfindungsgemäße phospholipidische Zusammensetzung) zum Lösungsmittel zwischen 7:3 bis 9:1 ausgewählt.

Wie bereits vorstehend im Zusammenhang mit der erfindungsgemäßen phospholipidischen Zusammensetzung dargelegt wurde, eignet sich die erfindungsgemäße phospholipidische Zusammensetzung insbesondere auch als Zusatzstoff bei der Herstellung von Lebensmitteln, Genußmitteln, Kosmetika oder pharmazeutischen Zubereitungen. Hierbei fallen unter den Begriff "Genußmittel" alle solche Produkte, die für den menschlichen Verzehr geeignet sind, wie beispielsweise Schokolade, Kuchen, Backmischungen, Mixgetränke o. dgl., während unter den Begriff "Kosmetika" alle solche Mittel verstanden werden, die das menschliche Äußere verändern, so insbesondere Hautpflegemittel, Mittel zur Hautbräunung, kosmetische Packungen, Desodoranten, Badezusätze, Parfums, Duftwässer, Lippenstifte, Lidschatten o.dgl..

Vorzugsweise wird die zuvor beschriebene erfindungsgemäße phospholipidische Zusammensetzung als Emulgator bzw. Dispergator in kosmetischen Zubereitungen, in Nahrungs- bzw. Genußmittel oder in pharmazeutischen Zubereitungen eingesetzt. Von daher betrifft die vorliegende Erfindung auch Nahrungsmittel, Genußmittel, kosmetische oder pharmazeutische Zubereitungen, die die eingangs beschriebenen unterschiedlichen Ausführungsformen der erfindungsgemäßen phospholipidischen Zusammensetzung enthalten.

Wird die erfindungsgemäße phospholipidische Zusammensetzung zur Herstellung von kosmetischen oder pharmazeutischen Zubereitungen verwendet, so kann eine derartige kosmetische bzw. pharmazeutische Zubereitung als flüssige, halbfeste oder feste Zubereitung vorliegen.

Für flüssige kosmetische oder pharmazeutische Zubereitungen kommen Tropfen, Tinkturen oder Sprays in Frage, die neben der erfindungsgemäßen phospholipidischen Zusammensetzung ggf. weitere Wirkstoffe enthalten, wobei diese weiteren Wirkstoffe in der flüssigen Zubereitung gelöst, suspendiert, emulgiert oder dispergiert sind.

Als halbfeste kosmetische bzw. pharmazeutische Zubereitungen kommen beispielsweise Gele, Salben, Cremes oder Schäume in Frage, während unter feste Zubereitungen beispielsweise Pulver, Puder, Granulate, Pellets oder Mikrokapseln fallen.

Wird die eingangs beschriebene phospholipidische Zusammensetzung in Form einer flüssigen Darreichungsform als pharmazeutisches und/oder kosmetisches Mittel angeboten, so empfiehlt es sich, hierfür möglichst solche Verdünnungsmittel zu verwenden, die die Haut bei einer topischen Anwendung nicht reizen. Dies trifft beispielsweise auf Wasser, einwertige Alkohole, vorzugsweise Ethanol, Isopropanol oder n-Propanol, mehrwertige Alkohole, insbesondere Glycerin und/oder Propandiol, Polyglykole, insbesondere Polyethylenglykol, Polypropylenglykol und/oder Miglyol, Glycerinformal, Dimethylisosorbit, natürliche und synthetische Öle und/oder Ester, zu.

Für die Herstellung von halbfesten Darreichungsformen, wie beispielsweise Gele, Salben, Cremes und Schäume eignen sich neben den zuvor genannten Verdünnungsmitteln zusätzlich noch Grundmassen, wie beispielsweise Bentonit, Veegum, Guarmehl und/oder Cellulosederivate, insbesondere Methylcellulose und/oder Carboxymethylcellulose. Ebenso kommen als Grundmasse anstelle der zuvor genannten Grundmassen oder zusätzlich zu den zuvor genannten Grundmassen Polymere aus Vinylalkohole, Vinylpyrolidone, Alginate, Pektine, Polyacrylate, feste und/oder flüssige Polyethylenglykole, Paraffine, Fettalkohole, Vaseline, Wachse, Fettsäuren und/oder Fettsäureester in Frage.

Für die Herstellung von festen, ebenfalls zur topischen Anwendung geeigneten Zubereitungen, wie beispielsweise die zuvor genannten Pulver, Puder, Granulate, Pellets oder Mikrokapseln, besteht die Möglichkeit, hier als Bindemittel beispielsweise kolloidale Kieselsäure, Talkum, Milchzucker, Stärkepulver, Zucker, Cellulosederivate, Gelatine, Metalloxide und/oder Metallsalze zu verwenden, wobei die Konzentration der erfindungsgemäßen phospholipidischen Zusammensetzung in derartigen festen pharmazeutischen und/oder kosmetischen Zubereitungen davon abhängt, wofür diese Zubereitungen eingesetzt werden.

Darüber hinaus kann eine unter Verwendung der erfindungsgemäßen phospholipidischen Zusammensetzung hergestellte kosmetische und/oder pharmazeutische Zusammensetzung noch weitere Bestandteile, wie beispielsweise gezielte Wirkstoffe, Konservierungsmittel, Stabilisatoren, Tenside, Emulgatoren, Penetrationsförderer, Spreitungsmittel und/oder Treibmittel, enthalten.

Vorteilhafte Weiterbildungen der erfindungsgemäßen phospholipidischen Zusammensetzung sowie des erfindungsgemäßen Verfahrens sind in den Unteransprüchen angegeben.

Die erfindungsgemäße phospholipidische Zusammensetzung und das erfindungsgemäße Verfahren werden nachfolgend anhand von Ausführungsbeispielen näher erläutert.

**Ausführungsbeispiel 1**

500 g acetyliertes Rohphosphatid, das durch Acetylierung nach PARDUN (Die Pflanzenlecithine, Verlag für chemische Industrie H. Ziolkowsky KG, Augsburg, 1988) aus Soja-Rohlecithinen hergestellt wurde, wurde unter Rühren (Rührwerk: Stephan UC 5) bei 30 °C während 2 Stunden mit 1500 g Methanol behandelt. Anschließend wurde das gerührte Produkt eine Stunde stehengelassen bis ein Rückstand separiert war. Die Methanolphase wurde abdekantiert und anschließend wurde in einem Rotationsverdampfer die Flüssigkeit entfernt, wobei 120 g phospholipidische Zusammensetzung gewonnen wurde.

Die Konzentrationen an Phosphatidylcholin, N-Acetyl-Phosphatidylethanolamin und Phosphatidylethanolamin wurden in der phospholipidischen Zusammensetzung quantitativ bestimmt. Diese Analyse ergab folgende Werte:

29,9 Gew.% Phosphatidylcholin;

27 Gew.% N-Acetylphosphatidylethanolamin;

0,1 Gew.% Phosphatidylethanolamin;

43 Gew.% übliche Inhaltsstoffe.

Qualitativ konnten als übliche Inhaltsstoffe Triglyceride, freie Fettsäuren, Sterine, Tocopherole, Glykolipide, Kohlehydrate sowie sonstige Phospholipide ermittelt werden.

120 g der phospholipidischen Zusammensetzung wurden in 24 g Propylenglykol gelöst und die so hergestellte Lösung wurde als Lösung I bezeichnet.

**Ausführungsbeispiel 2**

500 g acetyliertes Rohphosphatid, das, wie in Ausführungsbeispiel 1 beschrieben, hergestellt wurde, wurde entsprechend dem Ausführungsbeispiel 1 behandelt. Abweichend hiervon wurde jedoch zur Isolierung der phospholipidischen Zusammensetzung 2.000 g Methanol eingesetzt, wobei die Isolierung in zwei Stufen durchgeführt wurde, wobei in jeder Stufe jeweils mit 1.000 kg Methanol gearbeitet wurde. Die Temperatur bei der Isolierung betrug 15 - 20 °C. Ansonsten entsprach das Vorgehen dem Vorgehen, wie dies in Ausführungsbeispiel 1 beschrieben ist.

Nach Abdampfen des Methanols betrug die Ausbeute (phospholipidische Zusammensetzung) 150 g.

In den vereinigten phospholipidischen Zusammensetzungen wurden quantitativ die Konzentrationen an Phosphatidylcholin, N-Acetylphosphatidylethanolamin sowie Phosphatidylethanolamin ermittelt. Die bei der Isolierung anfallenden üblichen Inhaltsstoffe wurden qualitativ untersucht.

Die in der vorstehenden Weise aus dem Methanolextrakt isolierte Fraktion wies folgende Zusammensetzung auf:

24,5 Gew.% Phosphatidylcholin;

22 Gew.% N-Acetylphosphatidylethanolamin;

0,5 Gew.% Phosphatidylethanolamin;

53 Gew.% übliche Inhaltsstoffe.

Die qualitative Zusammensetzung der üblichen Inhaltsstoffe unterschied sich nicht von der qualitativen Zusammensetzung der üblichen Inhaltsstoffe aus Beispiel 1.

150 g der so isolierten Fraktion wurden in 30 g Propylenglykol gelöst, wobei diese Lösung als Lösung II bezeichnet wurde.

**Ausführungsbeispiel 3**

Die Isolierung der phospholipidischen Zusammensetzung wurde so durchgeführt, wie dies in Beispiel 1 beschrieben ist. Abweichend hiervon wurden jedoch 2.500 g Methanol bei 20 - 25 °C eingesetzt.

Der vom Methanol befreite Extrakt wog 168 g. In diesem isolierten Extrakt wurden quantitativ die Konzentrationen an Phosphatidylcholin, N-Acetylphosphatidylethanolamin und Phosphatidylethanolamin bestimmt. Die desweiteren im isolierten Extrakt enthaltenen üblichen Bestandteile wurden qualitativ untersucht. Auch hier unterschieden sich die üblichen Bestandteile in ihrer qualitativen Zusammensetzung nicht von den üblichen Bestandteilen, wie diese nach Ausführungsbeispiel 1 isoliert worden waren.

Der isolierte, methanollösliche Extrakt wies folgende quantitative Zusammensetzung auf:

21 Gew.% Phosphatidylcholin;

20,5 Gew.% N-Acetylphosphatidylethanolamin;

1,5 Gew.% Phosphatidylethanolamin;

57 Gew.% übliche Inhaltsstoffe.

168 g des nach Ausführungsbeispiel 3 isolierten Extraktes wurden in 34 g Propylenglykol gelöst. Die so hergestellte Lösung wurde als Lösung III bezeichnet.

Anwendungsbeispiel A

Um das Emulgiervermögen der nach den Ausführungsbeispielen 1 bis 3 hergestellten Lösungen I bis III im Vergleich zu Rohlecithin (Lösung V) und acyliertem Rohlecithin (Lösung IV) zu überprüfen, wurde folgender Versuch durchgeführt:

Es wurde zunächst eine Fettmischung aus folgenden Stoffen hergestellt:

69,9 Gew.% Rindertalg

30,0 Gew.% Kokosöl (Cocopur, Fa. Rau)

0,1 Gew.% Sudanrot (Fa. Fluka)

Die oben genannten Stoffe wurden bei 70 °C im Wasserbad geschmolzen und so lange gerührt, bis sich der Farbstoff gleichmäßig in der Fettmischung verteilt hatte.

25,7 g der aufgeschmolzenen Fettmischung wurden mit 110 g Magermilchpulver (sprühgetrockneter Formtyp) und 1,76 g phospholipidischer Lösung (wahlweise Lösung I, Lösung II, Lösung III, Lösung IV und Lösung V) homogen vermischt. Diese Fett/Milch/Phospholipid-Mischung wurde mit einem Rührwerk (Krups-Mixer) mit 1 l Wasser (Temperatur 50 °C, 10 - 15 °dH) 4 Minuten wie folgt verrührt:

1 Minute Stufe I

1 Minute Stufe II

2 Minuten Stufe III

Die so erhaltene Emulsion wurde in einen auf 50 °C vorgeheizten Meßzylinder gefüllt, wobei zum Zeitpunkt des Auffüllens eine homogene Emulsion bestand.

Die Höhe der Aufrahmung der rot gefärbten Fettphasen wurde nach 30 Minuten gemessen und in mm ausgewiesen. Das Volumen der verwendeten Meßzylinder betrug 1 l, wobei alle Meßzylinder einen identisch großen Durchmesser aufwiesen.

Nach einer Verweilzeit von 30 Minuten ergaben sich die in der Tabelle 1 aufgeführten Dicken der aufgerahmten Fettphasen.

## Tabelle 1

Dicke der Fettphase in mm nach einer Verweilzeit von 30 Minuten

| Emulgiermittel | Dicke der Fettphase in mm |
|---|---|
| Lösung I (hergestellt nach Beispiel 1) | 3 |
| Lösung II (hergestellt nach Beispiel 2) | 4 |
| Lösung III (hergestellt nach Beispiel 3) | 7 |
| Lösung IV (*) | 14 |
| Lösung V (**) | 21 |

* und ** Vergleichsemulgatoren,

* 12 g acetyliertes Rohlecithin (Ausgangsprodukt bei den Beispielen 1 - 3), aufgenommen in 2,4 g Propylenglykol
** 12 g Rohlecithin (Sojabohne), aufgenommen in 2,4 g
Propylenglykol

Die vorstehenden Werte aus der Tabelle 1 belegen eindeutig, daß die Lösungen IV und V wesentlich schlechtere Emulgiereigenschaften aufweisen als die Lösungen I bis III.

Anwendungsbeispiel B

Um zu beweisen, daß die nach den Ausführungsbeispielen 1 bis 3 hergestellten phospholipidischen Zusammensetzungen hervorragende Emulgier- bzw. Dispergiereigenschaften aufweisen, wurden unter-

EP 0 604 806 A2

schiedliche Aromen, Riechstoffe und Lichtschutzfilter unter Verwendung der vorstehend beschriebenen Lösungen I bis V emulgiert bzw. dispergiert.

Hierbei wurde von folgender Grundrezeptur ausgegangen:

5 g der zu emulgierenden bzw. zu dispergierenden Substanz;

4 g wahlweise Lösung I bis V.

Die zuvor genannten Bestandteile wurden unter Rühren zusammengegeben. Anschließend wurden die vermischten Bestandteile in einem schnell laufenden Rührer (Waring Blendor) mit 100 g demineralisiertem Wasser 7 Minuten homogenisiert.

Von den Dispersionen bzw. Emulsionen wurde die Teilchengröße nach dem Prinzip des "Laser Light Scattering" (Coulter N4SD) bestimmt.

Die Stabilität der hergestellten Dispersionen bzw. Emulsionen wurde visuell ermittelt, wobei die nachfolgende Tabelle 2 die Zeiten angibt, nach denen die entsprechende Emulsionen bzw. nicht mehr stabil war bzw. nach denen der Versuch abgebrochen wurde.

Die Ergebnisse dieser Messung sind in der nachfolgenden Tabelle 2 wiedergegeben.

Tabelle 2

| zu emulgierende/dispergierende Substanz | | Teilchengröße in nm | Stabilität |
|---|---|---|---|
| Parfumkonzentrat 1 (Hersteller 1) | Lösung IV | 193 | nach 3 Tagen Separation |
| Parfumkonzentrat 1 (Hersteller 1) | Lösung III | 113 | nach 27 Tagen stabil; Versuch abgebrochen |
| Parfumkonzentrat 2 (Hersteller 2) | Lösung IV | 188 | nach 4 Tagen Separation |
| Parfumkonzentrat 2 (Hersteller 2) | Lösung I | 127 | nach 27 Tagen stabil; Versuch abgebrochen |
| Lichtfilter (Parsol MCX) | Lösung IV | 323 | nach 3 Tagen Separation |
| Lichtfilter (Parsol MCX) | Lösung III | 294 | nach 27 Tagen stabil; Versuch abgebrochen |
| Lichtfilter (Eusolex 4360) | Lösung IV | 360 | nach 3 Tagen Separation |
| Lichtfilter (Eusolex 4360) | Lösung III | 294 | nach 27 Tagen stabil; nach 29 Tagen erkennbare Separation |
| Lichtfilter (Eusolex 4360) | Lösung II | 272 | nach 45 Tagen stabil; Versuch abgebrochen |

Anwendungsbeispiel C

Es wurde ein Sonnenschutzgel C gemäß der nachfolgend wiedergegebenen Rezeptur hergestellt:

| | |
|---|---|
| 1 Octyl Methoxycinnamate | 2,5 % |
| 2 Benzophenone-3 | 0,5 % |
| 3 Distelöl | 10,0 % |
| 4 phospholipidische Zusammensetzung gemäß Beispiel 1 | 6,0 % |
| 5 Wasser, dem. | 79,0 % |
| 6 Hostacerin PN 73 | 1,0 % |
| 7 Parfumkonzentrat | 0,5 % |
| 8 Phenonip | 0,5 % |

Bei der Herstellung des Sonnenschutzgels wurden die Produkte 1 bis 3 unter Erwärmung zu einer klaren Lösung gebracht. Zu dieser klaren Lösung wurde das Produkt 4 bei gleicher Temperatur zugegeben. Anschließend wurde das Wasser (Produkt 5) auf die entsprechende Temperatur erwärmt und vorgelegt und in dieses Wasser wurden die vermischten Produkte 1 bis 4 eingerührt. Die Temperatur während des Dispergierens betrug 60 bis 70 °C. Die entstehende Dispersion wurde so lange gerührt, bis eine mittlere Teilchengröße von etwa 250 nm erreicht wurde. Hiernach wurde die Dispersion auf Raumtemperatur abgekühlt und mit Hostacerin (Produkt 6), dem Parfumkonzentrat (Produkt 7) und dem Konservierungsmittel (Produkt 8) unter Ausbildung des Sonnenschutzgels C vermischt.

10

Ausführungsbeispiel D

Es wurde ein Sonnenschutzgel D aus den nachfolgend wiedergegebenen Bestandteilen formuliert, wobei die Herstellung dieses Sonnenschutzgels so erfolgte, wie dies vorstehend für das Sonnenschutzgels C beschrieben ist.

Das Sonnenschutzgel D wies folgende Bestandteile auf:

| | |
|---|---|
| 1 Octyl Methoxycinnamate | 4,0 % |
| 2 Benzophenone-3 | 1,0 % |
| 3 Miglyol 812 | 10,0 % |
| 4 phospholipidische Zusammensetzung gemäß Beispiel 2 | 5,0 % |
| 5 Wasser, dem. | 77,3 % |
| 6 Xanthan | 1,7 % |
| 7 Phenonip | 0,5 % |
| 8 Parfumkonzentrat | 0,5 % |

Anwendungsbeispiel E

Es wurde eine Pre Shave Lotion E hergestellt, wobei die Pre Shave Lotion folgende Inhaltsstoffe aufwies:

| | |
|---|---|
| 1 phospholipidische Zusammensetzung gemäß Beispiel 3 | 0,5 % |
| 2 Alpha-Tocopherol | 0,5 % |
| 3 Parfumöl | 0,25 % |
| 4 Carbopol 980 | 0,75 % |
| 5 NaOH, 10%ig | q.s. |
| 6 Ethanol | 15,0 % |
| 7 Wasser, dem. | 82,0 % |
| 8 Phenonip | 1,0 % |

Die Produkte 1 bis 3 wurden vorgemischt und mit einem Drittel der Gesamtmenge Wasser versetzt und so lange gerührt, bis eine Dispersion entstand, die eine mittlere Teilchengröße von ca. 200 nm aufwies. Diese Dispersion wurde in das Produkt 4 eingearbeitet. Nach Ausquellung wurde das restliche Wasser zugesetzt und der pH-Wert der Lotion wurde mit dem Produkt 5 auf 6,8 eingestellt. Hiernach wurden die Produkte 6 und 8 zugerührt.

Anwendungsbeispiel F

Es wurde eine Sonnenschutzlotion F aus den folgenden Bestandteilen hergestellt:

| 1 Crodamol DOA | 8,0 % |
|---|---|
| 2 Cithrol GMS/AS | 6,0 % |
| 3 Crodamol PMP | 5,0 % |
| 4 Base CB 3929 | 5,0 % |
| 5 Antaron V-220 | 2,0 % |
| 6 Emulgin B 2 | 1,2 % |
| 7 Emulgin B 1 | 0,8 % |
| 8 Carbopol 980 | 0,1 % |
| 9 Wasser, dem. | 4,9 % |
| 10 phospholipidische Zusammensetzung gemäß Beispiel 2 | 6,0 % |
| 11 Octyl Methoxycinnamate | 6,0 % |
| 12 Propylenglykol | 2,0 % |
| 13 Aloe vera Gel 2/912800 | 2,0 % |
| 14 Wasser, dem. | 47,0 % |
| 15 Panthenol 50 P | 2,0 % |
| 16 Parfumöl Lafetto 100.034 | 0,3 % |
| 17 Euxyl K 400 | 0,1 % |
| 18 Triethanolamin 10%ig | 1,6 % |

Zur Herstellung wurden zunächst die Produkte 10, 11 und 14 dispergiert bis eine mittlere Teilchengröße von ca. 200 nm entstand. Zu dieser Dispersion wurden die Produkte 12 und 13 sowie das aus den Produkten 8 und 9 bestehende Gel zugesetzt.

Die Phase wurde dann zu der aus den Produkten 1 bis 7 bestehenden aufgeschmolzenen und homogenisierten Phase zugeführt. Die vereinigten Phasen wurden unter Rühren auf eine Temperatur von etwa 35 °C abgekühlt. Das dabei entstehende Produkt wurde mit den Produkten 15, 16, 17 und 18 versetzt und unter Rühren auf Raumtemperatur abgekühlt.

Anwendungsbeispiel G

Es wurde eine Pflegecreme G aus den nachfolgend aufgeführten Bestandteilen hergestellt:

| 1 Emulgin B1 | 2,0 % |
|---|---|
| 2 Syncrowax BB 4 | 2,0 % |
| 3 Crodawax GP 200 | 3,0 % |
| 4 Cithrol GMS/AS | 4,0 % |
| 5 Dow DC 345 | 5,0 % |
| 6 Promyristyl PM 3 | 10,0 % |
| 7 Miglyol 812 | 10,0 % |
| 8 phospholipidische Zusammensetzung gemäß Beispiel 2 | 4,0 % |
| 9 Wasser, dem. | 57,7 % |
| 10 Propylenglykol | 2,0 % |
| 11 Parfumöl | 0,2 % |
| 12 Euxyl K 400 | 0,1 % |

Zur Herstellung der Pflegecreme G wurden die Produkte 8 bis 10 bei 70 °C dispergiert. Zu dieser Dispersion wurden bei 70 °C die aus den Produkten 1 bis 7 bestehende homogenisierte Phase zugesetzt, wobei zur Herstellung dieser homogenisierten Phase die Produkte 1 bis 7 bei 70 °C aufgeschmolzen und miteinander vermischt wurden. Die so hergestellte Mischung wurde unter Rühren auf etwa 40 °C abgekühlt und bei dieser Temperatur mit den Produkten 11 und 12 versetzt. Anschließend wurde die entstandene Creme unter Rühren auf Raumtemperatur abgekühlt.

Um zu überprüfen, ob die zuvor unter den Anwendungsbeispielen C bis G beschriebenen Zubereitungen Hautreizungen hervorrufen, wurde eine Untersuchung an Probanden durchgeführt.

Zu diesem Zweck wurden für jede Zubereitung 50 Probanden ausgewählt, die aufgrund der Aussage der Probanden besonders empfindlich auf kosmetische Zubereitungen reagieren.

Bei jedem Proband wurde auf eine lokalisierte Stelle am Unterarm (Fläche etwa 4 cm$^2$) 5 mal mit einem zeitlichen Abstand von 2 Stunden jeweils 0,5 g einer jeden Zubereitung aufgetragen. Vor dem erneuten Auftragen der Zubereitung wurde visuell beurteilt, ob eine Hautreizung auftrat. Die Ergebnisse dieser Untersuchung sind in der folgenden Tabelle 3 wiedergegeben.

Tabelle 3

| Untersuchung der Hautreizung, pro Zubereitung - 50 Probanden | | | | | |
|---|---|---|---|---|---|
| Zubereitung gemäß Anwendungsbeispiel | Anzahl der Probanden mit Hautreizung nach dem Auftragen | | | | |
| | einmal | zweimal | dreimal | viermal | fünfmal |
| C | 0 | 0 | 0 | 1 | 2 |
| D | 0 | 0 | 0 | 0 | 1 |
| E | 0 | 0 | 0 | 0 | 0 |
| F | 0 | 0 | 0 | 0 | 1 |
| G | 0 | 0 | 0 | 0 | 0 |

Desweiteren wurde die zuvor in Tabelle 3 aufgeführten Zubereitungen einem Alterungstest unterworfen. Bei diesem Alterungstest wurden die Zubereitungen bei einer relativen Luftfeuchtigkeit von 65 % bei einer Raumtemperatur von 40 °C während 30 Tage gelagert. Während der Lagerzeit und nach Ablauf der 30 Tage wurde der Geruch und die Konsistenz überprüft, ohne daß hierbei Veränderungen festgestellt werden konnten.

**Patentansprüche**

1. Phospholipidische Zusammensetzung mit einem Gehalt von mindestens 20 Gew.% Phosphatidylcholin, dadurch gekennzeichnet, daß die Zusammensetzung neben den üblichen Inhaltsstoffen desweiteren mindestens 20 Gew.% N-Acyl-Phosphatidylethanolamin sowie weniger als 3 Gew.% Phosphatidylethanolamin enthält.

2. Zusammensetzung nach Anspruch 1, dadurch gekennzeichnet, daß die Zusammensetzung zwischen 22 Gew.% und 40 Gew.%, insbesondere zwischen 25 Gew.% und 30 Gew.%, Phosphatidylcholin aufweist.

3. Zusammensetzung nach einem der vorangehenden Ansprüche, dadurch gekennzeichnet, daß die Zusammensetzung zwischen 21 Gew.% und 40 Gew.%, insbesondere zwischen 24 Gew.% und 28 Gew.%, N-Acyl-Phosphatidylethanolamin enthält.

4. Zusammensetzung nach einem der vorangehenden Ansprüche, dadurch gekennzeichnet, daß die Zusammensetzung zwischen 0,01 Gew.% und 1,5 Gew.% Phosphatidylethanolamin aufweist.

5. Zusammensetzung nach einem der vorangehenden Ansprüche, dadurch gekennzeichnet, daß die Zusammensetzung mindestens 30 Gew.% negativ geladene Phospholipide aufweist.

6. Zusammensetzung nach einem der vorangehenden Ansprüche, dadurch gekennzeichnet, daß die Zusammensetzung

25 - 30 Gew.% Phosphatidylcholin,
24 - 28 Gew.% N-Acyl-Phosphatidylethanolamin,
0,01 - 1,5 Gew.% Phosphatidylethanolamin, und
40,5 - 50,99 Gew.% sonstige Inhaltsstoffe

aufweist.

7. Zusammensetzung nach einem der vorangehenden Ansprüche, dadurch gekennzeichnet, daß das N-Acyl-Phosphatidylethanolamin N-Oleyl-Phosphatidylethanolamin, N-Palmitoyl-Phosphatidylethanolamin, N-Stearyl-Phosphatidylethanolamin, N-Myristoyl-Phosphatdiylethanolamin und/oder N-Acetyl-Phosphati-

EP 0 604 806 A2

dylethanolamin jeweils allein oder in Mischung enthält.

8. Zusammensetzung nach Anspruch 7, dadurch gekennzeichnet, daß das N-Acyl-Phosphatidylethanolamin zumindestens aus 90 Gew.% N-Acetyl-Phosphatidylethanolamin besteht.

9. Zusammensetzung nach einem der vorangehenden Ansprüche, dadurch gekennzeichnet, daß die Zusammensetzung in einem Lösungsmittel unter Ausbildung einer flüssigen Zusammensetzung aufgenommen ist.

10. Zusammensetzung nach Anspruch 9, dadurch gekennzeichnet, daß die flüssige Zusammensetzung 70 Gew.% bis 90 Gew.% der festen phospholipidischen Zusammensetzung und als Lösungsmittel 30 Gew.% bis 10 Gew.% Propylenglykol enthält.

11. Verfahren zur Herstellung der phospholipidischen Zusammensetzung nach einem der vorangehenden Ansprüche, dadurch gekennzeichnet, daß als Ausgangssubstanz acyliertes Rohphosphatid eingesetzt wird und daß das acylierte Rohphosphatid mit Methanol behandelt und der in Methanol lösliche Anteil isoliert wird.

12. Verfahren nach Anspruch 11, dadurch gekennzeichnet, daß die Behandlung bei einer Temperatur zwischen 5 °C und 35 °C durchgeführt wird.

13. Verfahren nach einem der Ansprüche 11 oder 12, dadurch gekennzeichnet, daß die Behandlung mit Methanol während 30 Minuten bis 3 Stunden durchgeführt wird.

14. Verfahren nach einem der Ansprüche 11 bis 13, dadurch gekennzeichnet, daß der in Methanol lösliche Anteil zur Isolierung der phospholipidischen Zusammensetzung bei Temperaturen zwischen 70 °C und 120 °C getrocknet wird.

15. Verfahren nach einem der Ansprüche 11 bis 14, dadurch gekennzeichnet, daß das Massenverhältnis vom acylierten Rohphosphatid zum Methanol zwischen 1:3 bis 1:5 variiert.

16. Verfahren nach einem der Ansprüche 11 bis 15, dadurch gekennzeichnet, daß der isolierte, methanollösliche Anteil nach Entfernung des Methanols in einem geeigneten Lösungsmittel, insbesondere Propylenglykol, gelöst wird.

17. Verwendung der phospholipidischen Zusammensetzung nach einem der Ansprüche 1 bis 10 als Zusatzstoff bei der Herstellung von Lebensmittel, Genußmitteln, Kosmetika und/oder pharmazeutischen Zubereitungen.

18. Verwendung der phospholipidischen Zusammensetzung nach einem der Ansprüche 1 bis 10 als Emulgator, Dispergator, Wirkstoff und/oder Alterungsinhibitor in kosmetischen Zubereitungen.

19. Verwendung der phospholipidischen Zusammensetzung nach einem der Ansprüche 1 bis 10 als Emulgator, Dispergator und/oder Alterungsinhibitor in Nahrungs- bzw. Genußmittel.

20. Verwendung der phospholipidischen Zusammensetzung nach einem der Ansprüche 1 bis 10 als Emulgator, Dispergator, Wirkstoff und/oder Alterungsinhibitor in pharmazeutischen Zubereitungen.

21. Verwendung der phospholipidischen Zusammensetzung nach einem der Ansprüche 1 bis 10 als Emulgator, Dispergator und/oder Alterungsinhibitor in Parfum oder Parfumvorprodukte.

22. Lebensmittel, dadurch gekennzeichnet, daß es als Emulgator, Dispergator und/oder Alterungsinhibitor eine phospholipidische Zusammensetzung enthält, die neben üblichen Inhaltsstoffen mindestens 20 Gew.% Phosphatidylcholin, mindestens 20 Gew.% N-Acyl-Phosphatidylethanolamin und weniger als 3 Gew.% Phosphatidylethanolamin aufweist.

23. Genußmittel, dadurch gekennzeichnet, daß es als Emulgator, Dispergator und/oder Alterungsinhibitor eine phospholipidische Zusammensetzung enthält, die neben üblichen Inhaltsstoffen mindestens 20

14

Gew.% Phosphatidylcholin, mindestens 20 Gew.% N-Acyl-Phosphatidylethanolamin und weniger als 3 Gew.% Phosphatidylethanolamin aufweist.

24. Kosmetische Zubereitung, dadurch gekennzeichnet, daß sie als Emulgator, Dispergator, Alterungsinhibitor und/oder als Wirkstoff eine phospholipidische Zusammensetzung enthält, die neben üblichen Inhaltsstoffen mindestens 20 Gew.% Phosphatidylcholin, mindestens 20 Gew.% N-Acyl-Phosphatidylethanolamin und weniger als 3 Gew.% Phosphatidylethanolamin aufweist.

25. Pharmazeutische Zubereitung, dadurch gekennzeichnet, daß sie als Emulgator, Dispergator, Alterungsinhibitor und/oder als Wirkstoff eine phospholipidische Zusammensetzung enthält, die neben üblichen Inhaltsstoffen mindestens 20 Gew.% Phosphatidylcholin, mindestens 20 Gew.% N-Acyl-Phosphatidylethanolamin sowie weniger als 3 Gew.% Phosphatidylethanolamin aufweist.